# EUROPEAN PATENT APPLICATION

(11) **EP 4 134 978 A1**
(43) Date of publication of application: **15.02.2023**
(21) Application number: 22189523.8
(22) Date of filing: 09.08.2022
(51) Int. Cl.: G16H 50/20, G16H 50/30, G16H 50/70, G16H 10/20, G16H 10/60

(54) **MEDICAL INFORMATION PROCESSING SYSTEM, MEDICAL INFORMATION PROCESSING METHOD, AND PROGRAM**

(30) Priority: 11.08.2021 JP 2021131303; 29.07.2022 JP 2022122133
(71) Applicant: Canon Medical Systems Corporation, Tochigi (JP)
(72) Inventor: SAKAGUCHI, Takuya, Tochigi (JP)
(74) Representative: Marks & Clerk LLP

(57) **Abstract**

A medical information processing system of an embodiment includes an acquisition unit, an estimation unit, and an output control unit. The acquisition unit acquires examination data showing medical examination results with respect to a medical treatment subject and reply data showing reply results of a medical examination by interview with respect to the medical treatment subject. The estimation unit estimates information on medical treatment of the medical treatment subject by inputting the examination data of the medical treatment subject to a first model and estimates information on medical treatment of the medical treatment subject by inputting the examination data and the reply data of the medical treatment subject to a second model. The output control unit outputs a first estimation result of the first model and a second estimation result of the second model via an output unit.

## Description

### FIELD

Embodiments disclosed in the present description and drawings relate to a medical information processing system, a medical information processing method, and a program.

### BACKGROUND

Although the replies of a patient in a medical examination by interview are important information for estimating the condition of the patient, this is unstable information that often fluctuates due to the patient's own sensitivity and feelings. Currently, medical staff assimilate and understand these fluctuations and use it for medical treatment. Meanwhile, automation of medical treatment using artificial intelligence (AI) is under review. However, instability of replies of a patient in medical examinations by interview may have undesirable effects on medical treatment using AI.

### SUMMARY

An object of embodiments disclosed in the present description and drawings is to treat a patient without being affected by instability of replies in a medical examination by interview. However, the object of the embodiments disclosed in the present description and drawings is not limited to the above object. An object corresponding to each effect according to each configuration represented in embodiments which will be described later can be positioned as another object.
(1) A medical information processing system of an embodiment includes an acquisition unit, an estimation unit, and an output control unit. The acquisition unit acquires examination data showing medical examination results with respect to a medical treatment subject and reply data showing reply results in medical examinations by interview with respect to the medical treatment subject. The estimation unit estimates information on medical treatment of the medical treatment subject by inputting the examination data of the medical treatment subject to a first model. The first model is a model trained on the basis of a first training data set in which information on medical treatment of a learning subject is associated with the examination data of the learning subject as a correct label. Further, the estimation unit estimates information on medical treatment of the medical treatment subject by inputting the examination data and the reply data of the medical treatment subject to a second model. The second model is a model trained on the basis of a second training data set in which the information on medical treatment of the learning subject is associated with the examination data and the reply data of the learning subject as a correct label. The output control unit outputs a first estimation result representing the information on medical treatment estimated using the first model and a second estimation result representing the information on medical treatment estimated using the second model via an output unit.
(2) The output unit may include a display. The output control unit may display the first estimation result and the second estimation result side by side on the display.
(3) The medical information processing system may further include a determination unit. The determination unit compares the first estimation result with the second estimation result to determine whether or not the first estimation result and the second estimation result match.
(4) The determination unit may calculate a similarity between the first estimation result and the second estimation result. Further, the determination unit may determine that the first estimation result and the second estimation result match if the similarity is equal to or greater than a threshold value and may determine that the first estimation result and the second estimation result do not match if the similarity is less than the threshold value.
(5) The output control unit may output an alert via the output unit if it is determined that the first estimation result and the second estimation result do not match.
(6) The estimation unit may weight the reply data of the medical treatment subject at the time of inputting the reply data of the medical treatment subject to the second model. Further, the estimation unit may estimate information on medical treatment of the medical treatment subject by inputting the weighted reply data and the examination data of the medical treatment subject to the second model.
(7) The estimation unit may repeatedly estimate information on medical treatment while changing a weighting factor. The determination unit may compare the second estimation results representing a plurality of pieces of information on medical treatment repeatedly estimated using the second model to determine whether or not the plurality of second estimation results match.
(8) The output control unit may output the second estimation result for each weighting factor via the output unit.
(9) The information on medical treatment may include estimating a disease of a patient.
(10) A plurality of machine learning models may be provided for ages of medical treatment subjects.
(11) A first model for children may be provided which is trained using training data in which information on medical treatment of a child under the age of 18 is associated with examination data of the child as a correct label, and a second model for children may be provided which is trained using training data in which the information on medical treatment of the child under the age of 18 is associated with reply data and examination data of the child as a correct label. A first model for adults may be provided which is trained using training data in which information on medical treatment of an adult between ages of 18 and 65 is associated with examination data of the adult as a correct label, and a second model for adults may be provided which is trained using training data in which the information on medical treatment of the adult between ages of 18 and 65 is associated with reply data and examination data of the adult as a correct label. A first model for the elderly may be provided which is trained using training data in which information on medical treatment of the elderly over the age of 65 is associated with examination data of the elderly as a correct label, and a second model for the elderly may be provided which is trained using training data in which the information on medical treatment of the elderly over the age of 65 is associated with reply data and examination data of the elderly as a correct label.
(12) The estimation unit may be configured to estimate the information on medical treatment of the medical treatment subject by inputting the examination data of the medical treatment subject to the first model for children, the first model for adults, or the first model for the elderly depending on the age of the medical treatment subject, and to estimate the information on medical treatment of the medical treatment subject by inputting the examination data and the reply data of the medical treatment subject to the second model for children, the second model for adults or the second model for the elderly depending on the age of the medical treatment subject.
(13) A plurality of machine learning models may be provided for onset periods of medical treatment subjects.
(14) A first model specialized for patients having onset periods of less than 1 year may be provided which is trained using training data in which information on medical treatment of a patient having an onset period of less than 1 year is associated with examination data of the patient as a correct label, and a second model specialized for patients having onset periods of less than 1 year may be provided which is trained using training data in which information on medical treatment of a patient having an onset period of less than 1 year is associated with reply data and examination data of the patient as a correct label. A first model specialized for patients having onset periods of 1 year or longer may be provided which is trained using training data in which information on medical treatment of a patient having an onset period of 1 year or longer is associated with examination data of the patient as a correct label, and a second model specialized for patients having onset periods of 1 year or longer may be provided which is trained using training data in which information on medical treatment of a patient having an onset period of 1 year or longer is associated with reply data and examination data of the patient as a correct label.
(15) The estimation unit may be configured to estimate the information on medical treatment of the medical treatment subject by inputting the examination data of the medical treatment subject to the first model specialized for patients having onset periods of less than 1 year or the first model specialized for patients having onset periods of 1 year or longer depending on the onset period of the medical treatment subject, and to estimate the information on medical treatment of the medical treatment subject by inputting the examination data and the reply data of the medical treatment subject to the second model specialized for patients having onset periods of less than 1 year or the second model specialized for patients having onset periods of 1 year or longer depending on the onset period of the medical treatment subject.
(16) A plurality of machine learning models may be provided for experiences of replying of medical treatment subjects in medical examinations by interview.
(17) A first model specialized for first-visit patients may be provided which is trained using training data in which information on medical treatment of a first-visit patient having no experience of replying to medical examinations by interview is associated with examination data of the patient as a correct label, and a second model specialized for first-visit patients may be provided which is trained using training data in which the information on medical treatment of the first-visit patient is associated with reply data and examination data of the first-visit patient as a correct label. A first model specialized for patients who attend second and subsequent medical examinations may be provided which is trained using training data in which information on medical treatment of a patient who has one or more experiences of replying to medical examinations by interview in second and subsequent medical examinations is associated with examination data of the patient as a correct label, and a second model specialized for patients who attend second and subsequent medical examinations may be provided which is trained using training data in which the information on medical treatment of the patient who has one or more experiences of replying to medical examinations by interview in second and subsequent medical examinations is associated with reply data and examination data of the patient as a correct label.
(18) The estimation unit may be configured to estimate the information on medical treatment of the medical treatment subject by inputting the examination data of the medical treatment subject to the first model specialized for first-visit patients or the first model specialized for patients who attend second and subsequent medical examinations depending on the experience of replying of the medical treatment subject in medical examinations by interview, and to estimate the information on medical treatment of the medical treatment subject by inputting the examination data and the reply data of the medical treatment subject to the second model specialized for first-visit patients or the second model specialized for patients who attend second and subsequent medical examinations depending on the experience of replying of the medical treatment subject in medical examinations by interview.
(19) A medical information processing method using a computer, including:
   acquiring examination data showing medical examination results with respect to a medical treatment subject and reply data showing reply results of a medical examination by interview with respect to the medical treatment subject;
   estimating information on medical treatment of the medical treatment subject by inputting the examination data of the medical treatment subject to a first model trained on the basis of a first training data set, the first training data set being a data set in which information on medical treatment of a learning subject is associated with the examination data of the learning subject as a correct label;
   estimating information on medical treatment of the medical treatment subject by inputting the examination data and the reply data of the medical treatment subject to a second model trained on the basis of a second training data set, the second training data set being a data set in which the information on medical treatment of the learning subject is associated with the examination data and the reply data of the learning subject as a correct label; and
   outputting a first estimation result representing the information on medical treatment estimated using the first model and a second estimation result representing the information on medical treatment estimated using the second model via an output unit.
(20) A program for causing a computer to execute:
   acquiring examination data showing medical examination results with respect to a medical treatment subject and reply data showing reply results of a medical examination by interview with respect to the medical treatment subject;
   estimating information on medical treatment of the medical treatment subject by inputting the examination data of the medical treatment subject to a first model trained on the basis of a first training data set, the first training data set being a data set in which information on medical treatment of a learning subject is associated with the examination data of the learning subject as a correct label;
   estimating information on medical treatment of the medical treatment subject by inputting the examination data and the reply data of the medical treatment subject to a second model trained on the basis of a second training data set, the second training data set being a data set in which the information on medical treatment of the learning subject is associated with the examination data and the reply data of the learning subject as a correct label; and
   outputting a first estimation result representing the information on medical treatment estimated using the first model and a second estimation result representing the information on medical treatment estimated using the second model via an output unit.

### BRIEF DESCRIPTION OF THE DRAWINGS

FIG. 1 is a diagram showing a configuration example of a medical information processing system in a first embodiment.
FIG. 2 is a diagram showing a configuration example of a user interface in the first embodiment.
FIG. 3 is a diagram showing a configuration example of a medical information processing device in the first embodiment.
FIG. 4 is a flowchart showing a flow of a series of processing of a processing circuit according to the first embodiment.
FIG. 5 is a diagram for describing reply data.
FIG. 6 is a diagram showing an example of a first model.
FIG. 7 is a diagram showing an example of a second model.
FIG. 8 is a diagram showing an example of a screen of a display.
FIG. 9 is a diagram showing a state of comparing estimation results.
FIG. 10 is a diagram showing another example of the screen of the display.
FIG. 11 is a diagram schematically showing weighting of reply data.
FIG. 12 is a diagram showing another example of the screen of the display.

### DETAILED DESCRIPTION

Hereinafter, a medical information processing system, a medical information processing method, and a program of embodiments will be described with reference to the drawings.

### (First embodiment)

### [Configuration of medical information processing system]

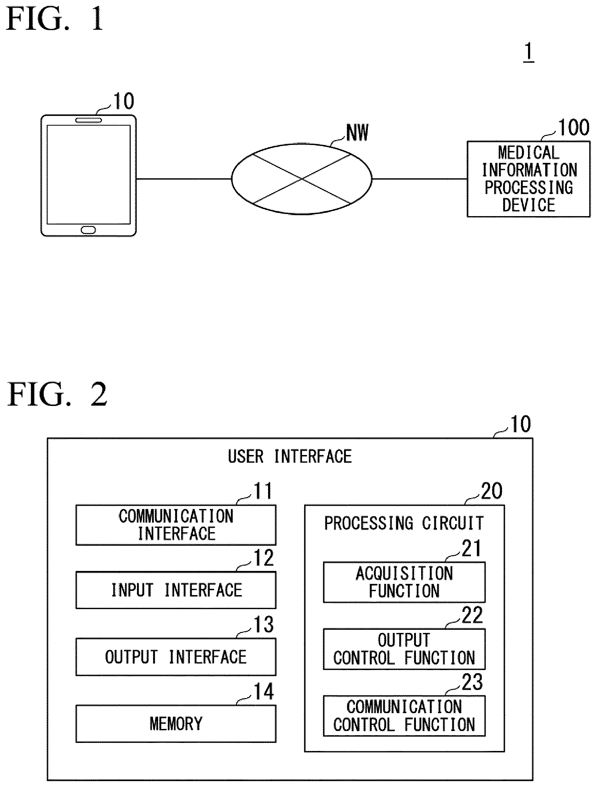

FIG. 1 is a diagram showing a configuration example of a medical information processing system 1 in a first embodiment. The medical information processing system 1 includes, for example, a user interface 10 and a medical information processing device 100. The user interface 10 and the medical information processing device 100 are connected such that they can communicate with each other via a communication network NW.

The communication network NW may mean an information communication network in general using telecommunications technology. For example, the communication network NW includes a telephone communication line network, an optical fiber communication network, a cable communication network, a satellite communication network, and the like in addition to a wireless/wired local area network (LAN) such as a hospital backbone LAN and the Internet.

The user interface 10 is used by patients and medical staff. For example, the user interface 10 is a touch interface or a voice user interface, and more specifically, a terminal device such as a personal computer, a tablet terminal, or a mobile phone. Medical staff are typically doctors but may be nurses or other people involved in medical treatment. For example, a patient inputs his/her own replies in a medical examination by interview to the user interface 10 through touch or voice input. A medical staff member may perform an oral medical examination by interview for the patient, listens to replies from the patient, and input listening comprehension results to the user interface 10.

In the present embodiment, "medical treatment" may include not only treatment such as surgery and medication but also examination up to treatment or after treatment and any other medical practices up to or after the treatment.

The user interface 10 transmits information input by a patient or a medical staff member to the medical information processing device 100 via the communication network NW or receives information from the medical information processing device 100.

The medical information processing device 100 receives information from the user interface 10 via the communication network NW and processes the received information. Then, the medical information processing device 100 transmits the processed information to the user interface 10 via the communication network NW. In addition to or instead of transmitting the processed information to the user interface 10, the medical information processing device 100 may transmit the processed information to a dedicated terminal of a medical staff member installed in a hospital.

The medical information processing device 100 may be a single device or may be a system in which a plurality of devices connected via the communication network NW operate in cooperation. That is, the medical information processing device 100 may be realized by a plurality of computers (processors) included in a distributed computing system or a cloud computing system. The medical information processing device 100 does not necessarily have to be a separate device from the user interface 10 and may be a device integrated with the user interface 10.

### [Configuration of terminal device]

FIG. 2 is a diagram showing a configuration example of the user interface 10 in the first embodiment. The user interface 10 includes, for example, a communication interface 11, an input interface 12, an output interface 13, a memory 14, and a processing circuit 20.

The communication interface 11 communicates with the medical information processing device 100 or the like via the communication network NW. The communication interface 11 includes, for example, a network interface card (NIC), an antenna for wireless communication, and the like.

The input interface 12 receives various input operations from an operator (for example, a patient), converts the received input operations into electrical signals, and outputs the electrical signals to the processing circuit 20. For example, the input interface 12 includes a mouse, a keyboard, a trackball, a switch, a button, a joystick, a touch panel, and the like. The input interface 12 may be, for example, a user interface that receives voice input, such as a microphone. When the input interface 12 is a touch panel, the input interface 12 may also have a display function of a display 13a included in the output interface 13 which will be described later.

In the present description, the input interface 12 is not limited to one including physical operation parts such as a mouse and a keyboard. For example, examples of the input interface 12 include an electrical signal processing circuit that receives an electrical signal corresponding to an input operation from an external input device provided separately from the device and outputs the electrical signal to a control circuit.

The output interface 13 includes, for example, the display 13a, a speaker 13b, and the like.

The display 13a displays various types of information. For example, the display 13a displays an image generated by the processing circuit 20, a graphical user interface (GUI) for receiving various input operations from an operator, and the like. For example, the display 13a is a liquid crystal display (LCD), a cathode ray tube (CRT) display, an organic electroluminescence (EL) display, or the like.

The speaker 13b outputs information input from the processing circuit 20 as voice information.

The memory 14 is realized by, for example, a semiconductor memory element such as a random access memory (RAM) or a flash memory, a hard disk, or an optical disc. These non-transient storage media may be realized by other storage devices connected via the communication network NW, such as a network attached storage (NAS) and an external storage server device. The memory 14 may include a non-transitory storage medium such as a read only memory (ROM) or a register.

The processing circuit 20 includes, for example, an acquisition function 21, an output control function 22, and a communication control function 23. The processing circuit 20 realizes these functions by, for example, a hardware processor (computer) executing a program stored in the memory 14 (storage circuit).

The hardware processor in the processing circuit 20 is defined as, for example, a circuit (circuitry) such as a central processing unit (CPU), a graphics processing unit (GPU), an application specific integrated circuit (ASIC), or a programmable logic device (for example, a simple programmable logic device (SPLD) or a complex programmable logic device (CPLD), or a field programmable gate array (FPGA)). Instead of storing the program in the memory 14, the program may be configured to be directly embedded in the circuit of the hardware processor. In this case, the hardware processor realizes the functions by reading and executing the program embedded in the circuit. The aforementioned program may be stored in the memory 14 in advance, or may be stored in a non-temporary storage medium such as a DVD or a CD-ROM and installed in the memory 14 from the non-temporary storage medium when the non-temporary storage medium is set in a drive device (not shown) of the user interface 10. The hardware processor is not limited to the one configured as a single circuit, and may be configured as one hardware processor by combining a plurality of independent circuits to realize each function. A plurality of components may be integrated into one hardware processor to realize each function.

The acquisition function 21 acquires input information via the input interface 12 or acquires information from the medical information processing device 100 via the communication interface 11.

The output control function 22 displays information acquired by the acquisition function 21 on the display 13a or outputs the information through the speaker 13b.

The communication control function 23 transmits information input to the input interface 12 to the medical information processing device 100 via the communication interface 11.

### [Configuration of medical information processing device]

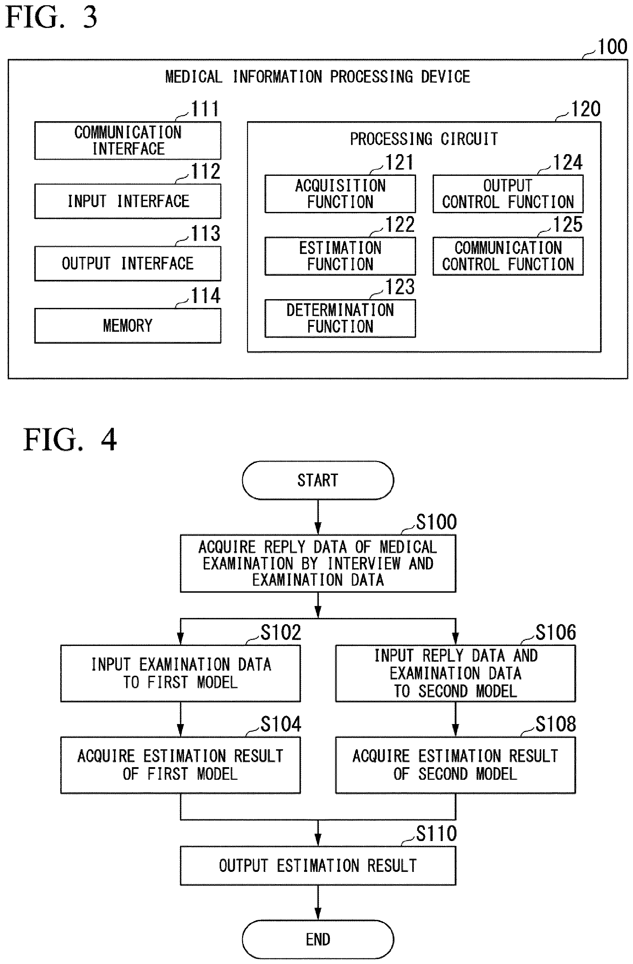

FIG. 3 is a diagram showing a configuration example of the medical information processing device 100 in the first embodiment. The medical information processing device 100 includes, for example, a communication interface 111, an input interface 112, an output interface 113, a memory 114, and a processing circuit 120.

The communication interface 111 communicates with the user interface 10 and the like via the communication network NW. The communication interface 111 includes, for example, an NIC or the like. The communication interface 111 is an example of an "output unit."

The input interface 112 receives various input operations from an operator, converts the received input operations into electrical signals, and outputs the electrical signals to the processing circuit 120. For example, the input interface 112 includes a mouse, a keyboard, a trackball, a switch, a button, a joystick, a touch panel, and the like. The input interface 112 may be, for example, a user interface that receives voice input, such as a microphone. When the input interface 112 is a touch panel, the input interface 112 may also have a display function of a display 113a included in the output interface 113 which will be described later.

In the present description, the input interface 112 is not limited to one provided with physical operating parts such as a mouse and a keyboard. For example, examples of the input interface 112 include an electrical signal processing circuit that receives an electrical signal corresponding to an input operation from an external input device provided separately from the device and outputs the electrical signal to a control circuit.

The output interface 113 includes, for example, the display 113a, a speaker 113b, and the like. The output interface 113 is another example of the "output unit".

The display 113a displays various types of information. For example, the display 113a displays an image generated by the processing circuit 120, a GUI for receiving various input operations from the operator, and the like. For example, the display 113a is an LCD, a CRT display, an organic EL display, or the like.

The speaker 113b outputs information input from the processing circuit 120 as voice information.

The memory 114 is realized by, for example, a semiconductor memory element such as a RAM or a flash memory, a hard disk, or an optical disc. These non-transient storage media may be realized by other storage devices connected via a communication network NW, such as a NAS or an external storage server device. The memory 114 may include a non-transitory storage medium such as a ROM or a register.

The memory 114 stores model information in addition to a program executed by a hardware processor. The model information is information (program or data structure) that defines a first model MDL1 and a second model MDL2. The first model MDL1 and the second model MDL2 may be implemented by a deep neural network (DNN) such as a convolutional neural network (CNN), for example. The first model MDL1 and the second model MDL2 are not limited to a DNN and may be implemented by other models such as a support vector machine, a decision tree, a naive Bayes classifier, and a random forest. Details of the first model MDL1 and the second model MDL2 will be described later.

When the first model MDL1 and the second model MDL2 are implemented by a DNN, the model information includes, for example, coupling information on how units included an input layer constituting the DNN, one or more hidden layers (intermediate layers), and an output layer are coupled with each other, and weight information on how many coupling coefficients are provided to data input/output between coupled units. The coupling information is, for example, information for specifying the number of units included in each layer, information for specifying the type of a unit that is a coupling destination of each unit, an activation function for realizing each unit, and a gate provided between units in a hidden layer. The activation function for realizing a unit may be, for example, a rectified linear unit (ReLU) function, an exponential linear units (ELU) function, a clipping function, a Sigmoid function, a step function, a hyperpolic tangent function, an equality function, or the like. The gate selectively passes or weights data transmitted between units, for example, depending on a value (e.g., 1 or 0) returned by the activation function. The coupling coefficients include, for example, a weight applied to output data when data is output from a unit of a certain layer to a unit of a deeper layer in a hidden layer of a neural network. The coupling coefficients may include a bias component specific to each layer, and the like.

The processing circuit 120 includes, for example, an acquisition function 121, an estimation function 122, a determination function 123, an output control function 124, and a communication control function 125. The acquisition function 121 is an example of an "acquisition unit," the estimation function 122 is an example of an "estimation unit," and the determination function 123 is an example of a "determination unit." The output control function 124 is an example of an "output control unit" and the communication control function 125 is another example of the "output control unit."

The processing circuit 120 realizes these functions by, for example, a hardware processor (computer) executing a program stored in the memory 114 (storage circuit).

The hardware processor in the processing circuit 120 means, for example, a circuit (circuitry) such as a CPU, a GPU, an application specific integrated circuit, or a programmable logic device (for example, a simple programmable logic device or a complex programmable logic device, or a field programmable gate array). Instead of storing the program in the memory 114, the program may be configured to be directly embedded in the circuit of the hardware processor. In this case, the hardware processor realizes the functions by reading and executing the program embedded in the circuit. The aforementioned program may be stored in the memory 114 in advance or may be stored in a non-temporary storage medium such as a DVD or a CD-ROM and installed in the memory 114 from the non-temporary storage medium when the non-temporary storage medium is set in a drive device (not shown) of the medical information processing device 100. The hardware processor is not limited to one configured as a single circuit and may be configured as one hardware processor by combining a plurality of independent circuits to realize each function. A plurality of components may be integrated into one hardware processor to realize each function.

### [Processing flow of medical information processing device]

Hereinafter, a series of processing performed by the processing circuit 120 of the medical information processing device 100 will be described with reference to a flowchart. FIG. 4 is a flowchart showing a flow of a series of processing of the processing circuit 120 according to the first embodiment.

First, the acquisition function 121 acquires reply data of a patient that is a medical treatment target (hereinafter, a medical treatment subject) for a medical examination by interview from the user interface 10 via the communication interface 111 and further acquires examination data of the medical treatment subject from examination equipment (step S100).

The examination equipment is equipment for medically examining patients and is, for example, an X-ray computed tomography (CT) device, a magnetic resonance imaging (MRI) device, a mammography device, a sound imaging diagnostic device, a nuclear medicine diagnostic device, a body fluid analysis device, devices for measuring vital signs, or the like. Examination data is quantitative digital data obtained by measuring biological information of the medical treatment subject by the above-mentioned various types of examination equipment. On the other hand, reply data is qualitative digital data including the subjectivity of the medical treatment subject. That is, the acquisition function 121 acquires quantitative data and qualitative data regarding the medical treatment subject.

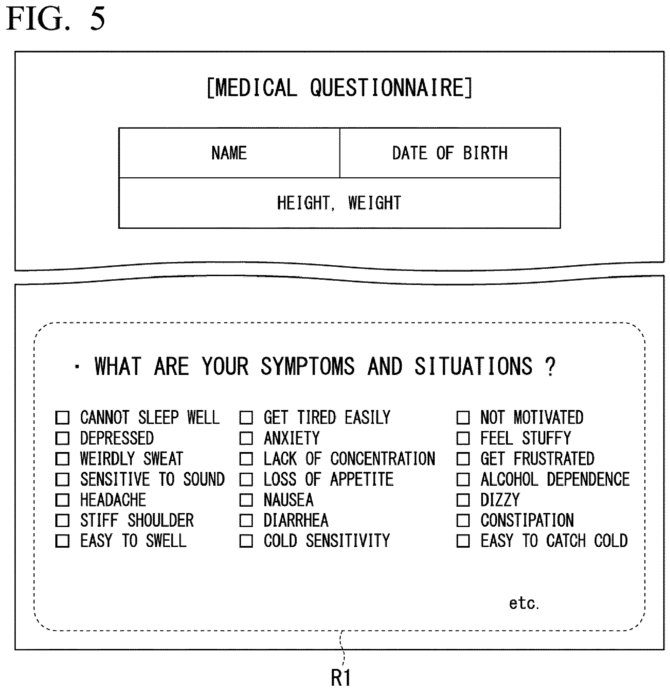

FIG. 5 is a diagram for describing reply data. For example, when a medical treatment subject visits a medical institution, he/she is required to fill necessary items (R1 in the figure) such as the current physical and mental conditions, a medical history, and presence/absence of allergies in a medical questionnaire as shown in the figure. The medical questionnaire may be displayed on the display 13a of the user interface 10 or may be printed on paper and distributed to the patient. When the patient has filled replies in the paper medical questionnaire, a medical staff member of the medical institution may input the fill-in content to the user interface 10. At this time, an optical character recognition/reader (OCR) may be used. The question content of the medical questionnaire may be output as voice from the speaker 113b of the user interface 10 or may be read aloud by a medical staff member of the medical institution. When the patient speaks replies to the user interface 10, the user interface 10 may obtain the replies spoken by the patient via a microphone. Alternatively or additionally, a medical staff member may listen to the replies spoken by the patient. When the medical staff member listens to the replies from the patient, the medical staff member may input listening comprehension results to the user interface 10. It is not necessary for questions to be determined in advance like a medical questionnaire, and a medical staff member may freely determine the contents of questions at the timing of examination. Replies in a medical examination by interview are medically called a chief complaint. Therefore, reply data may be replaced by chief complaint data.

Return to description of the flowchart of FIG. 4. When the reply data and the examination data regarding the medical examination by interview are acquired by the acquisition function 121, the estimation function 122 inputs the examination data of the medical treatment subject acquired by the acquisition function 121 to the first model MDL1 defined by model information stored in the memory 141 (step S102).

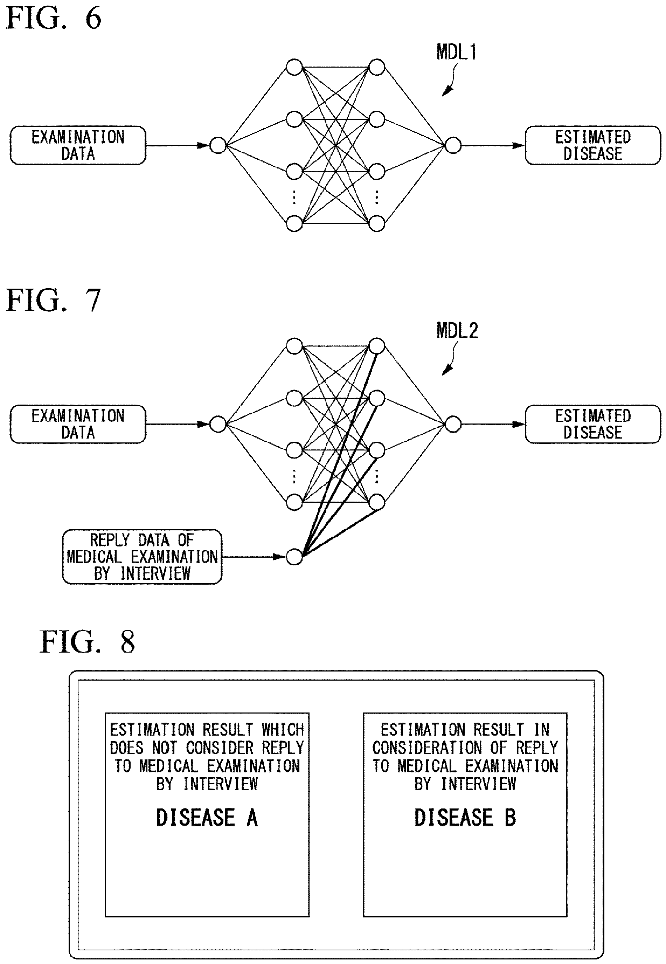

FIG. 6 is a diagram showing an example of the first model MDL1. The first model MDL1 is a machine learning model trained using a data set in which information on medical treatment of a learning subject is associated with examination data of the learning subject as a correct label (also referred to as a target) as training data. In other words, the first model MDL1 is a machine learning model trained to output information on medical treatment of a learning subject when examination data of the learning subject is input. The learning subject may be a patient who has been treated in the past. That is, the learning subject may be the same person as the medical treatment subject or may be a different person.

Information on medical treatment is, for example, information on medical treatment that should be taken after a starting point when a time at which the learning subject undergoes an examination is assumed as the starting point. Information on medical treatment is typically, but not limited to, estimating a disease that the patient has already suffered at the starting point or a disease that the patient is highly likely to suffer in the future. For example, information on medical treatment may include estimating or determining the name and type of an examination, prescription drug name, treatment policy, whether a patient can return home, the type of a patient's room, presence or absence of help of other staff, and the like. That is, information on medical treatment may include all matters in the future determined by medical examinations by interview. In the following description, as an example, it is assumed that information on medical treatment is "disease estimation."

When the information on medical treatment is "disease estimation," training data for learning the first model MDL1 is a data set in which a disease that a learning subject has already suffered from or a disease that the learning subject is highly likely to suffer in the future is associated with examination data of the learning subject as a correct label.

When examination data of a certain patient is input, as illustrated, the first model MDL1 trained using such training data outputs, as an estimation result, the disease of the patient (an example of "information on medical treatment"). The estimation result of the first model MDL1 is represented by, for example, a multidimensional vector or tensor. The vector or tensor includes the probability of being a disease as an element value. For example, it is assumed that there are a total of three types of diseases that the medical treatment subjects can suffer: disease A, disease B, and disease C. In this case, the vector or tensor can be represented as (e1, e2, e3) where the probability of disease A is e1, the probability of disease B is e2, and the probability of disease C is e3.

Return to description of the flowchart in FIG. 4. Next, the estimation function 122 acquires the estimation result of the disease from the first model MDL1 to which the examination data of the medical treatment subject has been input (step S104). The estimation result includes a disease that is estimated to be already afflicted by the medical treatment subject or a disease that is estimated to be afflicted by the medical treatment subject in the future.

On the other hand, when the reply data and the examination data for the medical examination by interview are acquired by the acquisition function 121, the estimation function 122 inputs the reply data and the examination data of the medical treatment subject acquired by the acquisition function 121 to the second model MDL2 defined by the model information stored in the memory 141 (step S106).

FIG. 7 is a diagram showing an example of the second model MDL2. The second model MDL2 is a machine learning model trained using a data set in which information on medical treatment of a learning subject is associated with reply data and examination data of the learning subject as a correct label as training data. In other words, the second model MDL2 is a machine learning model trained to output information on medical treatment of a learning subject when reply data and examination data of the learning subject are input.

Information on medical treatment here is, for example, information on medical treatment that should be taken after a starting point when a time at which a learning subject undergoes an examination or a time at which the learning subject replies to a medical examination by interview is assumed as the starting point. As described above, information on medical treatment is typically, but not limited to, estimating a disease that the patient has already suffered at the starting point or a disease that the patient is highly likely to suffer in the future, and may include all matters in the future determined by medical examinations by interview. In the following description, as an example, it is assumed that information on medical treatment is "disease estimation."

When information on medical treatment is "disease estimation," training data for learning the second model MDL2 is a data set in which a disease that a learning subject has already suffered or a disease that the learning subject is highly likely to suffer in the future is associated with reply data and examination data of the learning subject as a correct label.

When reply data and examination data of a certain patient are input, as illustrated, the second model MDL2 trained using such training data outputs, as an estimation result, the disease of the patient. The estimation result of the second model MDL2 may be represented by a multidimensional vector or tensor like the estimation result of the first model MDL1.

Return to description of the flowchart in FIG. 4. Next, the estimation function 122 acquires the estimation result of the disease from the second model MDL2 to which the reply data and the examination data of the medical treatment subject have been input (step S108). The estimation result also includes a disease that is estimated to be already afflicted by the medical treatment subject or a disease that is estimated to be afflicted by the medical treatment subject in the future.

Next, the output control function 124 outputs a first estimation result representing the disease of the medical treatment subject estimated using the first model MDL1 and a second estimation result representing the disease of the medical treatment subject estimated using the second model MDL2 via the output interface 113 (step S110). Accordingly, processing of this flowchart ends.

FIG. 8 is a diagram showing an example of a screen of the display 113a. As illustrated, for example, the output control function 124 may display the first estimation result and the second estimation result side by side on the display 113a. The illustrated example shows that the disease estimated using only the examination data without using the reply data for the medical examination by interview (that is, the first estimation result) is "disease A" and the disease estimated using both of the reply data and the examination data for the medical examination by interview (that is, the second estimation result) is "disease B." By performing such a display, it is possible to remind a medical staff member that, when reply data of a certain patient is used, the instability of the reply data may affect the estimation result of the disease. As a result, the medical staff member can suspect that the chief complaint of the patient may include instability such as fluctuation and thus can diagnose the patient more carefully as compared to a case in which the first and second estimation results are identical.

The communication control function 125 may transmit the first estimation result and the second estimation result to the user interface 10 via the communication interface 111. When the communication interface 11 receives the first estimation result and the second estimation result from the medical information processing device 100, the output control function 22 of the user interface 10 causes the display 13a of the output interface 13 to display the estimation results as an image or causes the estimation results to be output as voice through the speaker 13b.

According to the first embodiment described above, the processing circuit 120 of the medical information processing device 100 acquires examination data of a medical treatment subject and reply data for a medical examination by interview. The processing circuit 120 estimates information on medical treatment of the medical treatment subject by inputting the examination data of the medical treatment subject to the first model MDL1 trained in advance. For example, the processing circuit 120 may estimate the disease of the medical treatment subject as the information on medical treatment.

The processing circuit 120 estimates information on medical treatment of the medical treatment subject by inputting the examination data and the reply data of the medical treatment subject to the second model MDL2 trained in advance. For example, the processing circuit 120 may estimate the disease of the medical treatment subject as the information on medical treatment.

Then, the processing circuit 120 outputs the first estimation result representing the information on medical treatment (e.g., the disease of the medical treatment subject) estimated using the first model MDL1 and the second estimation result representing the information on medical treatment (e.g., the disease of the medical treatment subject) estimated using the second model MDL2 via the output interface 113. Accordingly, a medical staff member can treat the patient without being affected by instability of the replies of the patient in a medical examination by interview.

### (Second embodiment)

Hereinafter, a second embodiment will be described. The second embodiment differs from the first embodiment in that it is determined whether or not the first estimation result and the second estimation result match. Hereinafter, differences from the first embodiment will be mainly described, and the points common to the first embodiment will be omitted. In the description of the second embodiment, the same parts as those of the first embodiment will be described with the same reference numerals.

The determination function 123 of the second embodiment compares the first estimation result representing information on medical treatment (for example, the disease of the medical treatment subject) estimated using the first model MDL1 to the second estimation result representing information on medical treatment (for example, the disease of the medical treatment subject) estimated using the second model MDL2 and determines whether or not the estimation results match.

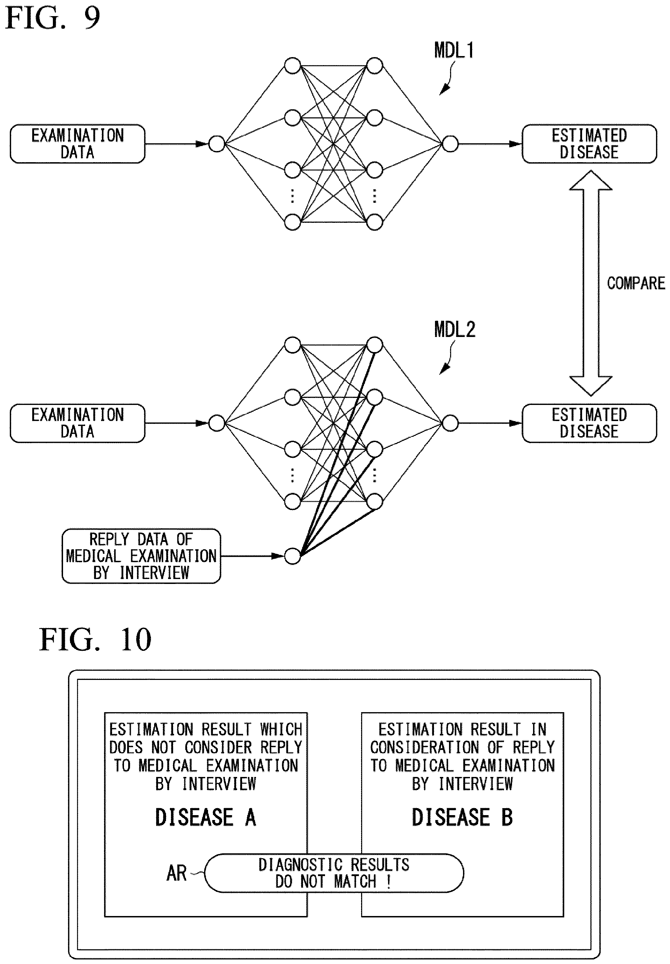

FIG. 9 is a diagram showing a state of comparing estimation results. For example, the determination function 123 compares the first estimation result and the second estimation result and calculates a similarity between the estimation results. For example, the determination function 123 may calculate a cosine similarity between a vector/tensor representing the first estimation result and a vector/tensor representing the second estimation result. Then, the determination function 123 determines that the first estimation result and the second estimation result match if the calculated similarity is equal to or greater than a threshold value and determines that the first estimation result and the second estimation result do not match if the similarity is less than the threshold value.

When the determination function 123 determines that the first estimation result and the second estimation result do not match, the output control function 124 of the second embodiment outputs an alert AR via the output interface 113 in order to notify a medical staff member that the estimation results do not match.

FIG. 10 is a diagram showing another example of the screen of the display 113a. For example, at the time of displaying the first estimation result and the second estimation result side by side on the display 113a, the output control function 124 may also display the alert AR if the estimation results do not match. The output control function 124 may output an alert sound via the speaker 113b.

According to the second embodiment described above, the processing circuit 120 compares the first estimation result and the second estimation result and determines whether or not the first estimation result and the second estimation result match. Upon determining that the first estimation result and the second estimation result do not match, the processing circuit 120 outputs the alert AR via the output interface 113. Accordingly, it is possible to more strongly remind a medical staff member that instability of reply data can affect disease estimation results.

### (Third embodiment)

Hereinafter, a third embodiment will be described. The third embodiment differs from the above-described embodiments in that, when information on medical treatment of a medical treatment subject is estimated by inputting examination data and reply data of the medical treatment subject to the second model MDL2 trained in advance, the reply data is weighted. Hereinafter, differences from the first embodiment and the second embodiment will be mainly described, and the points common to the first embodiment and the second embodiment will be omitted. In the description of the third embodiment, the same parts as those of the first embodiment or the second embodiment will be described with the same reference numerals.

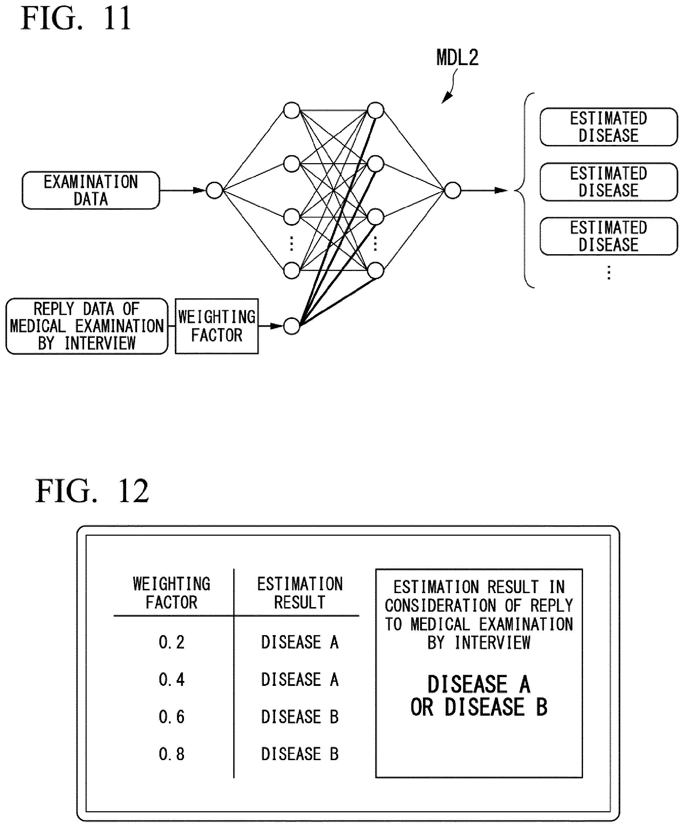

FIG. 11 is a diagram schematically showing weighting of reply data. The estimation function 122 of the third embodiment weights reply data of a medical treatment subject at the time of inputting the reply data to the second model MDL2 trained in advance. For example, the estimation function 122 determines a weighting factor in the range of 0.0 to 1.0, multiplies the reply data by the weighting factor, and then inputs the result to the second model MDL2. Accordingly, the degree to which the reply data contributes to the second estimation result can be changed according to the weighting factor. The smaller the weighting factor (closer to 0.0), the closer the second estimation result is expected to be to the first estimation result. That is, it is expected that the degree of similarity between the first estimation result and the second estimation result will increase.

The estimation function 122 repeats inputting the reply data to the second model MDL2 while changing the weighting factor. As a result, the second estimation result is obtained for each weighting factor.

The determination function 123 of the third embodiment compares a plurality of second estimation results obtained for respective weighting factors with each other and determines whether or not the second estimation results match each other.

The output control function 124 of the third embodiment displays, for example, a plurality of second estimation results obtained for respective weighting factors side by side on the display 113a. When the determination function 123 determines that the second estimation results do not match each other, the output control function 124 may also display the alert AR on the display 113a.

FIG. 12 is a diagram showing another example of the screen of the display 113a. As illustrated, the output control function 124 may display diseases estimated by the second model MDL2 side by side on the display 113a for respective weighting factors. The illustrated example shows that the result is "disease A" in the case of weighting factors of 0.2 and 0.4, whereas the result is "disease B" in the case of weighting factors of 0.6 and 0.8. In such a case, "disease A" and "disease B" may be conceived as candidates for diseases that can be afflicted by the medical treatment subject. Therefore, the output control function 124 may display two types of candidates, "disease A" and "disease B," on the display 113a.

According to the third embodiment described above, the processing circuit 120 weight reply data at the time of estimating information on medical treatment of a medical treatment subject by inputting examination data and the reply data of the medical treatment subject to the second model MDL2 trained in advance. The processing circuit 120 repeats estimating information on medical treatment while changing the weighting factor. The processing circuit 120 compares second estimation results respectively representing pieces of information on medical treatment repeatedly estimated using the second model MDL2 and determines whether or not the plurality of second estimation results match each other. Then, the processing circuit 120 outputs the second estimation result for each weighting factor. Accordingly, for example, if the second estimation result does not change even if the weighting factor is changed, it can be determined that the reply data of the medical treatment subject does not affect the estimation result of the information on medical treatment. That is, it is possible to accurately estimate the information on medical treatment even if the reply data of the medical treatment subject is used.

### (Fourth embodiment)

Hereinafter, a fourth embodiment will be described. The fourth embodiment differs from the above-described embodiments in that a weighting factor of reply data is determined on the basis of the age of a medical treatment subject, a period elapsed since the medical treatment subject suffered from a disease (onset period), the experience of replying of the medical treatment subject in a medical examination by interview, and the like. Hereinafter, differences from the first to third embodiments will be mainly described, and the points common to the first to third embodiments will be omitted. In description of the fourth embodiment, the same parts as those of the first to third embodiments will be described with the same reference numerals.

As in the third embodiment, the estimation function 122 of the fourth embodiment weights reply data of a medical treatment subject at the time of inputting the reply data of the medical treatment subject to the second model MDL2 trained in advance. At this time, the estimation function 122 of the fourth embodiment determines a weighting factor of the reply data on the basis of the age of the medical treatment subject, a period elapsed since the medical treatment subject suffered from a disease (onset period), the experience of replying of the medical treatment subject in a medical examination by interview, and the like.

For example, children and the elderly are more likely to have fluctuations in replying to medical examinations by interview than adults. Therefore, the estimation function 122 in the fourth embodiment may decrease the weighting factor of reply data as a medical treatment subject becomes younger than a certain reference age (for example, 18 years old). Similarly, the estimation function 122 may decrease the weighting factor of reply data as the medical treatment subject becomes older than a certain reference age (for example, 65 years).

A patient who has just suffered from a disease is more likely to have more fluctuations in replying to medical examinations by interview than patients who do not. Therefore, the estimation function 122 in the fourth embodiment may decrease the weighting factor of reply data as a period elapsed since a medical treatment subject suffered from a disease is shorter (shorter after suffering from the disease).

A patient who replies to a medical examination by interview for the first time at the first visit is more likely to have more fluctuations in replying to the medical examination by interview than experienced patients who have already been examined many times and replied to medical examinations by interviews. Therefore, the estimation function 122 in the fourth embodiment may decrease the weighting factor of reply data as the medical treatment subject has less experience in replying to medical examinations by interview.

Accordingly, it is possible to remove the influence of fluctuations in replies caused by the age, onset period, reply experience, and the like of a medical treatment subject from the output result of a trained model MDL. As a result, a medical staff member can further treat patients while considering the uncertainty of a trained model MDL.

### (Fifth embodiment)

Hereinafter, a fifth embodiment will be described. The fifth embodiment differs from the above-described embodiments in that a plurality of machine learning models are provided for ages of medical treatment subjects, a plurality of machine learning models are provided for onset periods of medical treatment subjects, and a plurality of machine learning models are provided for experiences of replying of medical treatment subjects in medical examinations by interview. Hereinafter, differences from the first to fourth embodiments will be mainly described, and the points common to the first to fourth embodiments will be omitted. In description of the fifth embodiment, the same parts as those of the first to fourth embodiments will be described with the same reference numerals.

For example, the processing circuit 120 may generate a first model MDL1 for children by using training data in which information on medical treatment (for example, a disease) of a child under the age of 18 is associated with examination data of the child as a correct label, and generate a second model MDL2 for children by using training data in which the information on medical treatment (for example, a disease) of the child under the age of 18 is associated with reply data and examination data of the child as a correct label.

Similarly, the processing circuit 120 may generate a first model MDL1 for adults by using training data in which information on medical treatment (for example, a disease) of an adult between ages of 18 and 65 is associated with examination data of the adult as a correct label, and generate a second model MDL2 for adults by using training data in which the information on medical treatment (for example, a disease) of the adult between ages of 18 and 65 is associated with reply data and examination data of the adult as a correct label.

The processing circuit 120 may generate a first model MDL1 for the elderly by using training data in which information on medical treatment (for example, a disease) of the elderly over the age of 65 is associated with examination data of the elderly as a correct label, and generate a second model MDL2 for the elderly by using training data in which the information on medical treatment (for example, a disease) of the elderly over the age of 65 is associated with reply data and examination data of the elderly as a correct label. Accordingly, it is possible to remove the influence of reply fluctuations due to an age from the uncertainty of a trained model MDL.

For example, the processing circuit 120 of the fifth embodiment may generate a first model MDL1 specialized for patients having onset periods of less than 1 year by using training data in which information on medical treatment (for example, a disease) of a patient having an onset period of less than 1 year is associated with examination data of the patient as a correct label, and generate a second model MDL2 specialized for patients having onset periods of less than 1 year by using training data in which information on medical treatment (for example, a disease) of a patient having an onset period of less than 1 year is associated with reply data and examination data of the patient as a correct label.

The processing circuit 120 may generate a first model MDL1 specialized for patients having onset periods of 1 year or longer by using training data in which information on medical treatment (for example, a disease) of a patient having an onset period of 1 year or longer is associated with examination data of the patient as a correct label, and generate a second model MDL2 specialized for patients having onset periods of 1 year or longer by using training data in which information on medical treatment (for example, a disease) of a patient having an onset period of 1 year or longer is associated with reply data and examination data of the patient as a correct label. Accordingly, it is possible to remove the influence of reply fluctuations due to an onset period from the uncertainty of a trained model MDL.

For example, the processing circuit 120 of the fifth embodiment may generate a first model MDL1 specialized for first-visit patients by using training data in which information on medical treatment (for example, a disease) of a first-visit patient having no experience of replying to medical examinations by interview is associated with examination data of the patient as a correct label, and generate a second model MDL2 specialized for first-visit patients by using training data in which the information on medical treatment (for example, a disease) of the first-visit patient is associated with reply data and examination data of the first-visit patient as a correct label.

For example, the processing circuit 120 of the fifth embodiment may generate a first model MDL1 specialized for patients who attend second and subsequent medical examinations by using training data in which information on medical treatment (for example, a disease) of a patient who has one or more experiences of replying to medical examinations by interview in second and subsequent medical examinations is associated with examination data of the patient as a correct label, and generate a second model MDL2 specialized for patients who attend second and subsequent medical examinations by using training data in which the information on medical treatment (for example, a disease) of the patient who has one or more experiences of replying to medical examinations by interview in second and subsequent medical examinations is associated with reply data and examination data of the patient as a correct label. Accordingly, it is possible to remove the influence of reply fluctuations due to experience of medical examination by interview from the uncertainty of a trained model MDL.

### (Other embodiments)

Hereinafter, other embodiments will be described. Although the first model MDL1 and the second model MDL2 have been described as different models in the above-described embodiments, the present invention is not limited thereto and they may be the same model. The first model MDL1 and the second model MDL2 may have some parameters (such as DNN weights and bias components) different from each other and the remaining parameters that are common.

Although the user interface 10 and the medical information processing device 100 have been described as different devices in the above-described embodiments, the present invention is not limited thereto. For example, the user interface 10 and the medical information processing device 100 may be a single integrated device. For example, the processing circuit 20 of the user interface 10 may further include the estimation function 122 and a determination function 123 included in the processing circuit 120 of the medical information processing device 100 in addition to the acquisition function 21, the output control function 22, and the communication control function 23. In this case, the user interface 10 can perform various types of processing of the flowcharts described above standalone (offline).

As also described in the above-described embodiments, information on medical treatment output by the first model MDL1 and the second model MDL2 is typically estimating a disease of a patient, but is not limited thereto and may include all matters in the future determined by medical examinations by interview, such as the name and type of an examination, the name of prescription drug, treatment policy, whether or not a patient can return home, the type of a patient's room, and whether or not there is help from other staff.

According to at least one embodiment described above, the processing circuit 120 of the medical information processing device 100 acquires examination data and reply data of a medical examination by interview of a medical treatment subject. The processing circuit 120 estimates information on medical treatment of the medical treatment subject by inputting the examination data of the medical treatment subject to the first model MDL1 trained in advance. For example, the processing circuit 120 estimates a disease of the medical treatment subject. The processing circuit 120 estimates information on medical treatment of the medical treatment subject by inputting the examination data and the reply data of the medical treatment subject to the second model MDL2 trained in advance. For example, the processing circuit 120 estimates a disease of the medical treatment subject. Then, the processing circuit 120 outputs the first estimation result representing the information on medical treatment estimated using the first model MDL1 (for example, a disease of the medical treatment subject) and the second estimation result representing the information on medical treatment estimated using the second model MDL2 (for example, a disease of the medical treatment subject) via the output interface 113. Accordingly, a medical staff member can treat patients without being affected by the instability of their replies in medical examinations by interview.

Although several embodiments have been described, these embodiments are presented as examples and are not intended to limit the scope of the invention. These embodiments can be implemented in various other embodiments, and various omissions, replacements, and changes can be made without departing from the scope of the invention as defined by the appended claims.

## Claims

1. A medical information processing system (1) comprising:
an acquisition unit (121) configured to acquire examination data showing medical examination results with respect to a medical treatment subject and reply data showing reply results of a medical examination by interview with respect to the medical treatment subject;
an estimation unit (122) configured to estimate information on medical treatment of the medical treatment subject by inputting the examination data of the medical treatment subject to a first model (MDL1) trained on the basis of a first training data set, and to estimate information on medical treatment of the medical treatment subject by inputting the examination data and the reply data of the medical treatment subject to a second model (MDL2) trained on the basis of a second training data set, the first training data set being a data set in which information on medical treatment of a learning subject is associated with the examination data of the learning subject as a correct label, the second training data set being a data set in which the information on medical treatment of the learning subject is associated with the examination data and the reply data of the learning subject as a correct label; and
an output control unit (124) configured to output a first estimation result representing the information on medical treatment estimated using the first model and a second estimation result representing the information on medical treatment estimated using the second model via an output unit (113).

2. The medical information processing system (1) according to claim 1, wherein the output unit (113) includes a display (113a), and the output control unit (124) is configured to display the first estimation result and the second estimation result side by side on the display (113a).

3. The medical information processing system (1) according to claim 1 or 2, further comprising: a determination unit (123) configured to compare the first estimation result with the second estimation result to determine whether or not the first estimation result and the second estimation result match.

4. The medical information processing system (1) according to claim 3, wherein the determination unit (123) is configured to calculate a similarity between the first estimation result and the second estimation result, determine that the first estimation result and the second estimation result match if the similarity is equal to or greater than a threshold value, and determine that the first estimation result and the second estimation result do not match if the similarity is less than the threshold value.

5. The medical information processing system (1) according to claim 3 or 4, wherein the output control unit (124) is configured to output an alert via the output unit (113) if it is determined that the first estimation result and the second estimation result do not match.

6. The medical information processing system (1) according to any one of claims 3 to 5, wherein the estimation unit (122) is configured to weight the reply data of the medical treatment subject at the time of inputting the reply data of the medical treatment subject to the second model, and estimate information on medical treatment of the medical treatment subject by inputting the weighted reply data and the examination data of the medical treatment subject to the second model.

7. The medical information processing system (1) according to claim 6,
wherein the estimation unit (122) is configured to repeatedly estimate information on medical treatment while changing a weighting factor; and
wherein the determination unit (123) is configured to compare the second estimation results representing a plurality of pieces of information on medical treatment repeatedly estimated using the second model to determine whether or not the plurality of second estimation results match.

8. The medical information processing system (1) according to claim 7, wherein the output control unit (124) is configured to output the second estimation result for each weighting factor via the output unit (113).

9. The medical information processing system (1) according to any preceding claim, wherein a plurality of machine learning models are provided for ages of medical treatment subjects.

10. The medical information processing system (1) according to claim 9, wherein a first model (MDL1) for children is provided which is trained using training data in which information on medical treatment of a child is associated with examination data of the child as a correct label, and a second model (MDL2) for children is provided which is trained using training data in which the information on medical treatment of the child is associated with reply data and examination data of the child as a correct label, wherein a first model (MDL1) for adults is provided which is trained using training data in which information on medical treatment of an adult is associated with examination data of the adult as a correct label, and a second model (MDL2) for adults is provided which is trained using training data in which the information on medical treatment of the adult is associated with reply data and examination data of the adult as a correct label, and wherein a first model (MDL1) for the elderly is provided which is trained using training data in which information on medical treatment of the elderly is associated with examination data of the elderly as a correct label, and a second model (MDL2) for the elderly is provided which is trained using training data in which the information on medical treatment of the elderly is associated with reply data and examination data of the elderly as a correct label.

11. The medical information processing system according to claim 10, wherein the estimation unit is configured to estimate the information on medical treatment of the medical treatment subject by inputting the examination data of the medical treatment subject to the first model for children, the first model for adults, or the first model for the elderly depending on the age of the medical treatment subject, and to estimate the information on medical treatment of the medical treatment subject by inputting the examination data and the reply data of the medical treatment subject to the second model for children, the second model for adults or the second model for the elderly depending on the age of the medical treatment subject.

12. The medical information processing system (1) according to any preceding claim, wherein a plurality of machine learning models are provided for onset periods of medical treatment subjects.

13. The medical information processing system (1) according to claim 12, wherein a first model (MDL1) specialized for patients having onset periods of less than 1 year is provided which is trained using training data in which information on medical treatment of a patient having an onset period of less than 1 year is associated with examination data of the patient as a correct label, and a second model (MDL2) specialized for patients having onset periods of less than 1 year is provided which is trained using training data in which information on medical treatment of a patient having an onset period of less than 1 year is associated with reply data and examination data of the patient as a correct label, and wherein a first model (MDL1) specialized for patients having onset periods of 1 year or longer is provided which is trained using training data in which information on medical treatment of a patient having an onset period of 1 year or longer is associated with examination data of the patient as a correct label, and a second model (MDL2) specialized for patients having onset periods of 1 year or longer is provided which is trained using training data in which information on medical treatment of a patient having an onset period of 1 year or longer is associated with reply data and examination data of the patient as a correct label.

14. A medical information processing method using a computer, comprising:
acquiring examination data showing medical examination results with respect to a medical treatment subject and reply data showing reply results of a medical examination by interview with respect to the medical treatment subject;
estimating information on medical treatment of the medical treatment subject by inputting the examination data of the medical treatment subject to a first model trained on the basis of a first training data set, the first training data set being a data set in which information on medical treatment of a learning subject is associated with the examination data of the learning subject as a correct label;
estimating information on medical treatment of the medical treatment subject by inputting the examination data and the reply data of the medical treatment subject to a second model trained on the basis of a second training data set, the second training data set being a data set in which the information on medical treatment of the learning subject is associated with the examination data and the reply data of the learning subject as a correct label; and
outputting a first estimation result representing the information on medical treatment estimated using the first model and a second estimation result representing the information on medical treatment estimated using the second model via an output unit.

15. A program for causing a computer to execute:
acquiring examination data showing medical examination results with respect to a medical treatment subject and reply data showing reply results of a medical examination by interview with respect to the medical treatment subject;
estimating information on medical treatment of the medical treatment subject by inputting the examination data of the medical treatment subject to a first model trained on the basis of a first training data set, the first training data set being a data set in which information on medical treatment of a learning subject is associated with the examination data of the learning subject as a correct label;
estimating information on medical treatment of the medical treatment subject by inputting the examination data and the reply data of the medical treatment subject to a second model trained on the basis of a second training data set, the second training data set being a data set in which the information on medical treatment of the learning subject is associated with the examination data and the reply data of the learning subject as a correct label; and
outputting a first estimation result representing the information on medical treatment estimated using the first model and a second estimation result representing the information on medical treatment estimated using the second model via an output unit.
